# EUROPEAN PATENT APPLICATION

(11) **EP 3 842 035 A1**
(43) Date of publication of application: **30.06.2021**
(21) Application number: 19219433.0
(22) Date of filing: 23.12.2019
(51) Int. Cl.: A61K 9/16, A61K 31/404, A61P 9/12, A61K 45/06

(54) **COMPOSITION FOR THE PREPARATION OF PERINDOPRIL ARGININE GRANULES, A METHOD FOR THEIR PREPARATION AND PHARMACEUTICAL COMPOSITION COMPRISING THE GRANULES**

(71) Applicant: KRKA, d.d., Novo mesto, 8000 Novo mesto (SI)
(72) Inventor: The designation of the inventor has not yet been filed
(74) Representative: Hoefer & Partner Patentanwälte mbB

(57) **Abstract**

The present invention relates to granules comprising perindopril arginine, as well as to a process for the preparation of granules comprising perindopril arginine. Furthermore, the present invention pertains to a pharmaceutical composition comprising granules comprising perindopril arginine.

## Description

### Field of the invention

The present invention relates to granules comprising perindopril arginine, a method for their preparation and pharmaceutical compositions comprising the granules.

### Background of the invention

Perindopril with its chemical name (2S,3aS,7aS)-1-((2S)-2-(((1S)-1-Ethoxycarbonyl)butyl)amino-1-oxopropyl)octahydro-1*H*-indole-2-carboxylic acid is described in EP49658.

Perindopril exists in different salt forms, wherein salts of perindopril exhibit polymorphism. Polymorphic forms of the t-butylamine salt of perindopril are described e.g. in EP1296947 (form alpha), EP1294689 (form beta), EP1296948 (form gamma). Perindopril arginine is known from e.g. EP1354873. This document also disclose that the arginine salt of perindopril is an alternate to tert-butylamine salt having different properties in terms of stability towards heat and humidity. Perindopril arginine can also be in crystalline or amorphous form. Crystaline forms are known e.g. from EP1989182 (form alpha; Servier), EP2016051 (form beta; Servier), EP2318365 (form gamma, Mylan), EP2612850 (form delta; Servier); amorphous perindopril arginine is known e.g. from EP2161257 (Apotex) and EP2682388 (Mylan).

Several pharmaceutical compositions comprising perindopril arginine are known in the prior art. For example, EP1989182, EP2016051 and EP2612850 describe tablet compositions comprising perindopril arginine, wherein compositions further comprise lactose. The same applies for the Coversyl Arginine® tablets present on the market. According to Coversyl Arginine® publicly available approval documents tablets are manufactured employing standard wet granulation techniques followed by film coating, however no process details are available for preparation of those tablets. Similar composition has been disclosed for combination product Coversyl Arginine Plus, wherein perindopril arginine is in combination with indapamide. WO2016/07163 and WO2017/032953 further describes fixed associations comprising perindopril arginine, e.g. combinations with amlodipine, indapamide, atorvastatin, bisoprolol, aspirin, or combinations thereof, wherein capsules comprising granules with perindopril arginine are disclosed.

Granules comprising perindopril arginine salt according to WO2017/032953 are consisted of hydroxyproylcellulose and sugar spheres, wherein their production comprises process wherein perindopril arginine and hydroxypropylcellulose are mixed in purified water, and then the suspension is sprayed onto sugar spheres to form the perindopril-minigranules.

Spraying of suspension onto sugar spheres requires longer process times, longer exposure of the active ingredient to process conditions, as well as higher consumption of energy, content of uniformity and processing into pharmaceutical composition.

It is therefore a problem underlying the present invention to provide alternative composition comprising perindopril arginine. In particular, it is an object to provide a composition for the preparation of perindopril arginine in the form of granules in which perindopril arginine is highly stable, especially it is an object to provide granules in which perindopril arginine is highly stable.

Another object of the present invention is to provide a method for the preparation of the granules which is simple and cost effective. Furthermore, it is an object of the present invention to provide granules comprising perindopril arginine having improved physical and mechanical properties while having fast and reliable dissolution of active ingredient, and which can be easily further processed into solid dosage forms such as capsules and/or tablets. Such granules can be stored for a long time alone as an intermediate product during the manufacture process, as well as they are stable in pharmaceutical composition alone or in combination with other active ingredients.

According to another object of the present invention is to provide pharmaceutical compositions comprising granules comprising perindopril arginine alone or in combination with other active ingredients.

Such granules also allows preparation of pharmaceutical compositions without lactose, therefore making available pharmaceutical compositions for people suffering of the lactose intolerance.

### Description of the invention

It is the object of the present invention to provide granules comprising perindopril arginine as the active ingredient, which are stable and which can be prepared by wet granulation.

It was found by the present inventors that this object can be achieved by granules comprising:
- perindopril arginine, in particular perindopril L-arginine,
- calcium chloride,
- at least one diluent and
- optionally at least one additional pharmaceutically acceptable excipient.

Granules according to the invention contain diluents such as cellulose, in particular microcrystalline cellulose, powdered cellulose, compressible sugar, fructose, dextranes, other sugars such as mannitol, sorbitol, lactitol, saccharose or a mixture thereof, siliconised microcrystalline cellulose, calcium hydrogen phosphate, calcium carbonate, calcium lactate or mixtures thereof. Preferably, the diluent is or includes microcrystalline cellulose.

Granules preferably comprise 4 to 25 % by weight, more preferably 10 to 15 % by weight of perindopril arginine, 0.5 - 5 % by weight, more preferably 1 to 3 % by weight of calcium chloride, 70 to 95 % by weight, more preferably 80 to 90 % by weight of diluent.

The term 'pharmaceutically acceptable excipient' has its normal meaning, i.e. it refers to any pharmaceutically acceptable substance that has no therapeutic activity as such but is present in the formulation for other reasons, e.g. to improve dissolution of the active ingredient itself. Pharmaceutically acceptable excipients may for instance be selected from fillers or carriers, binders, disintegrants, stabilizers, lubricants, glidants, surfactants, sweeteners, aromas, etc.

The granules of the present invention can be prepared by wet granulation, especially wet granulation using a granulation liquid comprising water.

Granules according to the present invention can be prepared in a process comprising
a. preparation of granulation liquid comprising perindopril arginine and calcium chloride, and optionally at least one pharmaceutically acceptable excipient,
b. preparation of granules comprising spraying granulation liquid on diluent, and optionally at least one pharmaceutically acceptable excipient, and
c. drying the granules.

Perindopril arginine (in particular perindopril L-arginine) used for preparation of granulation liquid can be in any polymorphic form or in amorphous form. The particle size of perindopril arginine used for preparation of granulation liquid can be in the range 0.1-2000 µm, preferably 50-1000 µm, in particular determined by a Malvern Mastersizer instrument based on laser diffraction.
In an embodiment, the particle size of perindopril arginine can be the average particle size. The term "average particle size" of perindopril arginine can in particular refer to the volume mean diameter of particles.

The granules of the present invention can comprise crystalline perindopril arginine, in particular perindopril arginine in a polymorphic form, or perindopril arginine in amorphous form. In particular, the perindopril arginine (especially as present in the granule) can be perindopril arginine comprising perindopril arginine amorphous form.

Especially, the granules of the present invention can comprise crystalline perindopril L-arginine, in particular perindopril L-arginine in a polymorphic form, or perindopril L-arginine in amorphous form. In particular, the perindopril L-arginine (especially as present in the granule) can be perindopril L-arginine comprising perindopril L-arginine amorphous form.

Preparation of granules is preferably performed in a high-shear mixer followed by drying, preferably by using fluid bed dryer.

Size of granules can be determined by sieve analysis according to method as determined in European Pharmacopeia (e.g. version 9.8, method 2.9.38) or by particle size measuring using a Retsch Particle Analyzer CAMSIZER XT. The particle size of granulate can be in the range 1-1000 µm, preferably 5-750 µm, most preferably 10-500 µm, in particular determined by a Retsch Particle Analyzer CAMSIZER XT.

According to another object of the present invention is to provide pharmaceutical compositions comprising
a.) granules comprising perindopril arginine (especially granules comprising perindopril arginine as described herein and/or in the claims annexed),
b.) optionally one or more additional active ingredients, and
c.) optionally one or more auxiliary pharmaceutically acceptable excipient. Optionally, the pharmaceutical composition can be a compressed pharmaceutical composition.

Active ingredients which can be optionally combined with granules comprising perindopril arginine to form pharmaceutical composition can be selected from the group, not limited, of indapamide, amlodipine, acetylsalicylic acid, bisoprolol, atorvastatine and rosuvastatine or pharmaceutically acceptable salts thereof. Pharmaceutical compositions comprising granules can be in a form of tablets or capsules, preferably in the form of tablets.

The term 'tablet' as used herein is intended to encompass compressed pharmaceutical dosage formulations of all shapes and sizes. The term 'capsule' encompasses pharmaceutical dosage form formulations wherein the active pharmaceutical ingredient, optionally mixed with further excipients, is enclosed in a capsule shell. Especially a pharmaceutical composition can be a composition in the form of a capsule, wherein granules comprising perindopril arginine as described herein and/or in the claims annexed are enclosed in a capsule shell.

The pharmaceutical formulation can be prepared by processes known to someone skilled in the field of pharmacy.

Tablets can be optionally coated by a film coating comprising water soluble polymer and at least one additional pharmaceutically acceptable excipient selected form plasticizers, pigments and/or colourants and antitacking agents. Water soluble polymer can be selected from low viscosity types of cellulose ethers such as hydroxypropyl cellulose, hydroxypropylmethyl cellulose and/or hydroxyethyl cellulose, polyvinyl alcohol and its copolymers such as block polymer of polyvinyl alcohol and polyethyleneglycole sold as Kollicoat® IR and Kollicoat® Protect. Pigments can be selected but are not limited to iron oxides and/or titanium dioxide. Plasticizers can be selected but are not limited to polyethileneglycole with molecular weight in the range from 200 to 10,000, propilenglycol and/or glycerol esters.

Film coating may optionally have a barrier function for moisture permeation.

Moreover, the granules of the present invention are for use in the treatment of cardiovascular diseases and cardiovascular conditions, especially for use in the treatment of hypertension and heart failure. Furthermore, the pharmaceutical composition of the present invention is for use in the treatment of cardiovascular diseases and cardiovascular conditions, especially for use in the treatment of hypertension and heart failure.

The following examples illustrate the invention and are not intended to restrict the scope of the invention in any way.

### Examples

### Example 1 - Preparation of granules comprising perindopril arginine

### Preparation of granulation liquid

Purified water (3494 g) was heated up to 40 °C in a mixer. CaCl₂ x 6H₂O (449 g; corresponding to 227.3 g of CaCl₂) was admixed to water, immediately followed by the addition of perindopril arginine (1869.05 g). Additional water (400 g) was added to rinse the equipment. The mixture was stirred until a clear pale yellow solution was obtained. The obtained solution was used as a granulation liquid.

### Preparation of granules

Microcrystalline cellulose (13674 g; Type 102) was homogenized in a high-shear mixer for 0.5 minutes, then the granulation liquid was sprayed at a rate of approximately 1500 g/min on the microcrystalline cellulose. The mixture was further kneaded for 1 minute.

The obtained granules were dried in a fluid bed dryer at a temperature between 50 and 65 °C until the water content was in the range 0.5 to 1.5 wt.-% (measured by loss on drying). The dried granules were then sieved through a mesh with screen size 18 # (mesh/inch). The composition of the perindopril arginine granules as prepared above described in Table 1, Example 1A. Examples 1B-D were prepared in analogous way.

**Table 1: Composition of the perindopril arginine granules**

| **Material** | **Example 1A** | **Example 1B** | **Example 1C** | **Example 1D** |
|---|---|---|---|---|
| Perindopril arginine | 5.000 mg | 5.000 mg | 5.000 mg | 5.000 mg |
| CaCl₂ | 0.608 mg | 0.908 mg | 0.508 mg | 1.108 mg |
| Cellulose, microcrystalline | 36.580 mg | 36.280 mg | 36.680 mg | 36.080 mg |
| **Total weight** | 42.188 mg | 42.188 mg | 42.188 mg | 42.188 mg |

The obtained perindopril arginine granules prepared according to Example 1A have the physical properties as described in Table 2.

**Table 2: Physical properties of the perindopril arginine granules**

| **Physical tests** | **Result** |
|---|---|
| Flow** time [s] | 25.3 |
| Angle of repose** [°] | 34.3 |
| Bulk volume [ml/g]* | 2.02 |
| Tapped volume [ml/g]* | 1.52 |
| Hausner ratio** | 1.33 |

**Table 3: Sieve analysis of the perindopril arginine granules**

| **Sieve analysis***** [wt.-%] | |
|---|---|
| <71 µm | 42 |
| <125 µm | 63 |
| <250 µm | 98 |
| <500 µm | 99.5 |

| | |
|---|---|
| Physical tests and Sieve analysis are performed according to Ph.Eur., version 9.8., following methods (*) Ph.Eur. 2.9.34, (**) Ph.Eur. 2.9.36 and (***) Ph.Eur. 2.9.38. | |

Table 4 shows the results of a particle size analysis, wherein the particle size was measured using a Retsch Particle Analyzer CAMSIZER XT.

**Table 4: Particle size analysis**

| | |
|---|---|
| D10c [µm] | 42,1 |
| D50c [µm] | 103,1 |
| D90c [µm] | 240,3 |
| **CEDc(vol) [µm]** | **129,5** |

The perindopril arginine in the granules according to Examples 1 are in amorphous form, which was confirmed by the X-ray powder diffraction pattern using X-ray diffractometer (PANalytical X'Pert PRO MPD, Almelo, NL) with Cu Kα radiation (λ = 1.5418 Å) at 45 kV and 40 mA and X' Celerator detector.

The perindopril arginine granules obtained according to Example 1 were produced in an easy and cost effective manner and showed excellent physical properties and stability after storage at 75% RH at temperatures of 40 °C. The granules were completely free of lactose and were suitable for pharmaceutical compositions for patients suffering lactose intolerance.

### Example 2 - Tablets comprising granules of perindopril arginine

The compositions of the tablets are disclosed in the following Table 5.

**Table 5: Compositions of 5 mg tablets**

| **Material** | **Example 2A** | **Example 2B** | **Example 2C** | **Example 2D** |
|---|---|---|---|---|
| Perindopril arginine granules | 42.188 mg | 42.188 mg | 42.188 mg | 42.188 mg |
| Cellulose, microcrystalline | 37.611 mg | 46,227 mg | 28.062 mg | 57.092 mg |
| Mannitol | 19.031 mg | / | / | / |
| Starch, Pregelatinized | / | 10.705 mg | 8.500 mg | / |
| Colloidal Silicon Dioxide (Aerosil 200 Pharma) | 0.270 mg | 0.430 mg | 0.350 mg | 0.270 mg |
| Magnesium Stearate | 0.900 mg | 0.450 mg | 0.900 mg | 0.450 mg |
| **Total weight of the tablet** | 100.000 mg | 100.000 mg | 80.000 mg | 100.000 mg |

### Preparation of compression mixture

The compression mixture used for forming the tablets was prepared by first mixing the granules as prepared in Example 1A with cellulose, microcrystalline and mannitol or starch, pregelatinized and colloidal silicon dioxide (see Table 5); subsequently, magnesium stearate was added.

### Compression

Compression was performed on a rotary tablet press at the speed of 44 rpm and force in the range of 2-4 kN, resulting in a tablet hardness of approx. 80 N.

Table 6 shows the results of the stability tests of the tablets according to Example 2D, at 40 °C at 1 and 3 months at 75 % RH, packed in Alu-Alu blisters. Results of perindopril hydrolytic product diketopiperazine (DKP) and total impurities are shown in table 6.

**Table 6: Stability tests**

| | Content of impurities | |
|---|---|---|
| Storing condition | Diketopiperazine imp. (DKP) | Total imp. |
| to | ≤ 0.10 % | 0.19 % |
| 40 °C / 75% RH 1m | ≤ 0.10 % | 0.23 % |
| 40 °C / 75% RH 3m | 0.21 % | 0.70 % |

HPLC is used to determine the purity. The tests are carried out on YMC Hydrosphere C18 (150 mm x 4,6 mm i.d., 3 µm particles) column. The mobile phase is a gradient of eluents A and B. (Eluent A: 0.01M sodium dihydrogen phosphate, pH adjusted to 2.0 with phosphoric acid : acetonitrile = 95 : 5 ; Eluent B: 0.01M sodium dihydrogen phosphate, pH adjusted to 2.0 with phosphoric acid : acetonitrile = 30 : 70) The chromatograph is equipped with a UV detector set at 210 nm, flow rate is 1.5 ml/min at 60°C. Impurities are calculated using external standard calibration.

Other strengths were prepared in an analogous manner, namely for 2.5 mg strength amounts of excipients were divided by two and for 10 mg strength amounts of excipients were doubled.

### Example 3 - Tablets comprising granules of perindopril arginine and indapamide

The compositions of the tablets are disclosed in the following Table 7.

**Table 7: Compositions of 5/1.25 mg tablets**

| **Material [mg]** | **5 / 1.25 mg** | **5 / 1.25 mg** | **5 / 1.25 mg** | **5 / 1.25 mg** | **5 / 1.25 mg** | **5 / 1.25 mg** | **5 / 1.25 mg** |
|---|---|---|---|---|---|---|---|
| Perindopril arginine granules | 42.188 | 42.188 | 42.188 | 42.188 | 42.188 | 42.188 | 42.188 |
| Coloidal silicon dioxide, hydrated (Syloid 244 FP EU) | 0.270 | 0.270 | 0.270 | 0.270 | 0.270 | 0.270 | 0.270 |
| Cellulose, microcrystalline, silicified LM | 55.342 | - | 27.671 | 44.966 | 44.966 | 39.966 | 39.966 |
| Mannitol | - | 55.342 | 27.671 | 10.376 | - | 10,376 | - |
| Pregelatinized starch (1500 LM) | - | - | - | - | 10.376 | - | 10,376 |
| Crosspovidone (Kolidon CL) | - | - | - | - | - | 5.000 | 5.000 |
| Indapamide (particle size d₉₀ 47 µm) | 1.250 | 1.250 | 1.250 | 1.250 | 1.250 | 1.250 | 1.250 |
| Sodium hydrogen carbonate | 0.500 | 0.500 | 0.500 | 0.500 | 0.500 | 0.500 | 0.500 |
| Magnesium Stearate | 0.450 | 0.450 | 0.450 | 0.450 | 0.450 | 0.450 | 0.450 |
| **Total weight of the tablet [mg]** | 100.000 | 100.000 | 100.000 | 100.000 | 100.000 | 100.000 | 100.000 |

### Preparation of compression mixture

The compression mixture was prepared by first mixing the granules as prepared in Example 1 with all excipients as disclosed in table 7, except magnesium stearate, and subsequently magnesium stearate was added.

### Compression

Compression was performed on a rotary tablet press at the speed of 60 rpm and force in the range of 5-6 kN, resulting in tablet hardness of approx. 90 N.

Other strengths were prepared in an analogous manner, namely for 10 / 2,5 mg strength amounts of excipients were doubled to a total weight of the tablet of 200 mg. Namely for 5 / 0,625 mg strength amounts of excipients were reduced by half to a total weight of the tablet of 50 mg.

### Example 4 - Tablets comprising granules of perindopril arginine and amlodipine

Table 8 shows the compositions of the tablets of Example 4.

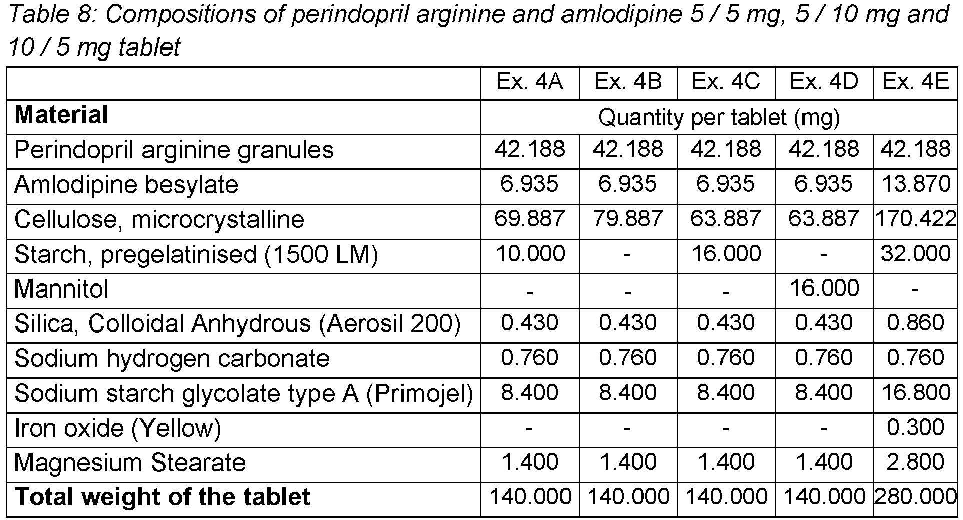

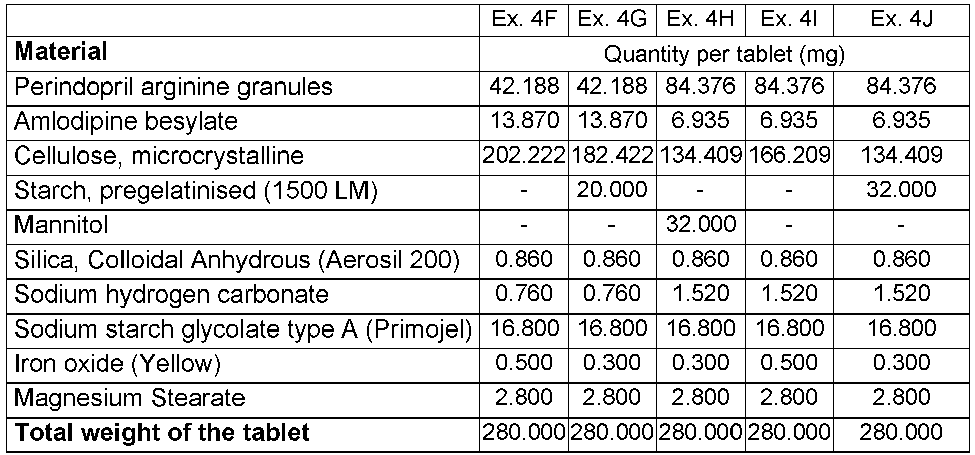

### Preparation of compression mixture

The compression mixture was prepared by first mixing the granules as prepared in Example 1 with all of the tablet components except for magnesium stearate. Then magnesium stearate was added. In order to achieve a homogenous distribution of the colour pigment, an additional premix with iron oxide and a portion of microcrystalline cellulose was passed through a sieve, if needed.

### Compression

Compression of 5/5 mg tablets was performed on a rotary tablet press at force in the range of 6-9 kN, resulting in tablet hardness of approx. 90 N.

Other strengths were prepared in an analogous manner, namely for 10/10 mg strength amounts of excipients were doubled according to 5/5 mg strength to a total weight of the tablet of 280 mg.

### Example 5 - Tablets comprising granules of perindopril arginine, indapamide and amlodipine

Table 9 shows the compositions of the tablets of Example 5.

**Table 9: Composition of Perindopril arginine, indapamide and amlodipine tablet**

| | **5a** | **5b** | **5c** | **5d** | **5e** | **5f** | **5g** | **5h** | **5i** |
|---|---|---|---|---|---|---|---|---|---|
| **Material** | | Quantity per tablet (mg) | | | | | | | |
| Perindopril arginine granules | 42.188 | 42.188 | 42.188 | 42.188 | 42.188 | 42.188 | 84.376 | 84.376 | 84.376 |
| Amlodipine besylate | 6.935 | 6.935 | 6.935 | 6.935 | 6.935 | 6.935 | 6.935 | 6.935 | 6.935 |
| Indapamide | 1.250 | 1.250 | 1.250 | 1.250 | 1.250 | 1.250 | 2.500 | 2.500 | 2.500 |
| Cellulose, microcrystalline, Type 112 | 67.637 | - | 70.637 | 47.637 | 87.797 | - | 122.209 | 141.209 | 148.069 |
| Cellulose, microcrystalline, silicified (PROSOLV SMCC) | - | 67.637 | - | - | - | - | - | - | - |
| Mannitol (Parteck M100) | - | - | - | 20.000 | - | - | - | - | - |
| Pregelatinized starch (Starch 1500 LM) | 11.000 | 11.000 | 8.000 | 11.000 | - | 11.000 | 42.000 | 20.000 | 17.000 |
| Sodium starch glycolate type A (Primojel) | 8.400 | 8.400 | 8.400 | 8.400 | - | 8.400 | 16.800 | 16.800 | 16.800 |
| Sodium Hydrogencarbon ate | 0.760 | 0.760 | 0.760 | 0.760 | - | 0.760 | 1.520 | 1.520 | 1.520 |
| Silica, Colloidal Hydrated (Syloid 244 FP EU) | 0.430 | 0.430 | 0.430 | 0.430 | 0.430 | - | 0.860 | 0.860 | 0.860 |
| Silica, Colloidal Anhydrous (Aerosil 200) | - | - | - | - | - | 0.430 | - | - | - |
| Iron oxide (Yellow) | - | - | - | - | - | - | - | - | 0.280 |
| Magnesium Stearate | 1.400 | 1.400 | 1.400 | 1.400 | 1.400 | 1.400 | 2.800 | 2.800 | 2.800 |
| **Total weight of the tablet** | **140.000** | **140.000** | **140.000** | **140.000** | **140.000** | **140.000** | **280.000** | **280.000** | **280.000** |

### Preparation of compression mixture

The compression mixture was prepared by first mixing granules as prepared in Example 1 with all of the tablet components except magnesium stearate. Then magnesium stearate was added.

### Compression

Compression of 5/5/1.25 mg tablets was performed on a rotary tablet press at force in the range of 6-7 kN, resulting in tablet strength of approx. 80 N.

Other strengths were prepared in an analogous manner, namely for 10/10/2.5 mg strength amounts of excipients were doubled to a total weight of the tablet of 280 mg, whereas for 10/5/1.25 mg strength less amlodipine besylate was replaced by microcrystalline cellulose (total weight of tablet was 280 mg).

Compression of 280 mg tablets was performed on a rotary tablet press at force in the range of 8.5 kN, resulting in tablet strength of approx. 100 N.

### Example 6 - Tablets comprising granules of perindopril arginine and bisoprolol

### fumarate

Table 10 shows the compositions of the tablets of Example 6.

**Table 10: Composition of bisoprolol fumarate and perindopril arginine 5/5 mg tablet**

| | Ex. 6A | Ex. 6B | Ex. 6C | Ex. 6D | Ex. 6E | Ex. 6F | Ex. 6G | Ex. 6H |
|---|---|---|---|---|---|---|---|---|
| **Material** | Quantity per tablet (mg) | | | | | | | |
| Perindopril arginine granules | 42.188 | 42.188 | 42.188 | 42.188 | 42.188 | 42.188 | 42.188 | 42.188 |
| Bisoprolol fumarate | 5.000 | 5.000 | 5.000 | 5.000 | 5.000 | 5.000 | 5.000 | 5.000 |
| Cellulose, microcrystalline | 39.372 | 34.372 | 48.372 | 54.672 | 20.672 | 34.372 | 20.672 | 54.672 |
| Starch, pregelatinised (1500 LM) | 9.000 | - | - | 13.700 | 13.700 | - | 13.700 | 13.700 |
| Calcium carbonate | 20.000 | 34.000 | 20.000 | - | 34.000 | 34.000 | 34.000 | - |
| Silica, Colloidal Anhydrous (Aerosil 200) | 0.240 | 0.240 | 0.240 | 0.240 | 0.240 | 0.240 | 0.240 | 0.240 |
| Sodium starch glycolate type A (Primojel) | 3.600 | - | 3.600 | 3.600 | 3.600 | 3.600 | - | - |
| Carmellose sodium (Ac-disol) | - | 3.600 | - | - | - | - | 3.600 | 3.600 |
| Magnesium Stearate | 0.600 | 0.600 | 0.600 | 0.600 | 0.600 | 0.600 | 0.600 | 0.600 |
| **Total weight of the tablet** | 120.000 | 120.000 | 120.000 | 120.000 | 120.000 | 120.000 | 120.000 | 120.000 |

### Preparation of compression mixture

The compression mixture was prepared by first mixing the granules as prepared in Example 1 with all of the tablet components except for magnesium stearate. Then magnesium stearate was added.

### Compression

Compression of 5/5 mg tablets was performed on a rotary tablet press at force in the range of 6-9 kN, resulting in tablet hardness of approx. 90 N.Other strengths were prepared in an analogous manner, namely for 10/10 mg strength amounts of excipients were doubled to a total weight of the tablet of 240 mg, whereas for 5/10 mg and 10/5 mg strength less bisoprolol fumarate and less perindopril arginine granules were replaced by microcrystalline cellulose (total weight of tablet was 240 mg).

### Example 7 - Tablets comprising an additional coating

In Example 7, the same tablets as obtained for Examples 6 were further coated.

| **Material** | Ex.7 |
|---|---|
| Bisoprolol fumarate / Perindopril arginine 5 / 5 mg core | 120.000 |
| Opadry® | 3.920 |
| Iron Oxide (Red) | 0.020 |
| Iron Oxide (Yellow) | 0.060 |
| Purified water | q.s. |
| **Total weight of the film coated tablet** | **124.000** |

Opadry®, Iron Oxide (Red) and Iron Oxide (Yellow) were dispersed in purified water. The tablets were coated with the obtained dispersion at temperature of outlet air being 40-48 °C.
Other strengths such as 5/10 mg, 10/5 mg and 10/10 mg were prepared in an analogous manner.

Throughout all Examples 2 to 7 the tablets showed excellent stability. More particular, the included perindopril arginine granules were physically and mechanically stable, did not show any decomposition, and showed good dissolution properties and availability. The physical properties were good. Moreover, the perindopril arginine granules and the tablets could be obtained easily in a cost effective manner without the use of expensive technical equipment. Before preparation of the tablets, the perindopril arginine granules could be stored for long time alone as an intermediate product without any negative side-effects. Also the compositions comprising perindopril arginine granules in combination with other active ingredients were stable. No lactose compounds were used for the preparation of the tablets, so that the tablets were suitable for patients suffering lactose intolerance.

## Claims

1. Granules comprising:
- perindopril arginine, in particular perindopril L-arginine,
- calcium chloride,
- at least one diluent, and
- optionally at least one additional pharmaceutically acceptable excipient.

2. The granules of claim 1, wherein the at least one diluent is or comprises a diluent selected from the group consisting of cellulose, in particular microcrystalline cellulose, powdered cellulose, compressible sugar, fructose, dextranes, other sugars such as mannitol, sorbitol, lactitol, saccharose or a mixture thereof, siliconised microcrystalline cellulose, calcium hydrogen phosphate, calcium carbonate, calcium lactate, and mixtures thereof.

3. The granules of any one of the preceding claims, wherein the at least one diluent is a diluent consisting of or comprising microcrystalline cellulose.

4. The granules of any one of the preceding claims, wherein the at least one additional pharmaceutically acceptable excipient is selected from the group consisting of fillers or carriers, binders, disintegrants, stabilizers, lubricants, glidants, surfactants, sweeteners, aromas, and mixtures thereof.

5. The granules of any one of the preceding claims, wherein the granules are granules comprising 4 to 25 % by weight, preferably 10 to 15 % by weight of perindopril arginine, 0.5 - 5 % by weight, preferably 1 to 3 % by weight of calcium chloride, 70 to 95 % by weight, preferably 80 to 90 % by weight of diluent, each relative to the total weight of the granules.

6. The granules of any one of the preceding claims, wherein the perindopril arginine is crystalline perindopril arginine, in particular perindopril arginine in a polymorphic form, or perindopril arginine in amorphous form, or
wherein the perindopril arginine is perindopril arginine comprising perindopril arginine amorphous form.

7. The granules of any one of the preceding claims, wherein the particle size of the granules is in the range of 1-1000 µm, preferably 5-750 µm, most preferably 10-500 µm, in particular determined by a Retsch Particle Analyzer CAMSIZER XT, and/or
wherein the granules are free of lactose, and/or
wherein the granules are granules prepared by wet granulation.

8. Process for the preparation of the granules according to any one of claims 1 to 7, said process comprising:
a. preparation of granulation liquid comprising perindopril arginine and calcium chloride, and optionally at least one pharmaceutically acceptable excipient,
b. preparation of granules comprising spraying granulation liquid on diluent, and optionally at least one pharmaceutically acceptable excipient, and
c. drying the granules.

9. The process of claim 8, wherein the particle size of the perindopril arginine used for the preparation of the granulation liquid is in the range of 0.1-2000 µm, preferably 50-1000 µm, in particular determined by a Malvern Mastersizer instrument based on laser diffraction.

10. The process of claim 8 or 9, wherein step b. is carried out in a high-shear mixer, and/or
wherein step c. is carried out in a fluid bed dryer.

11. The process of any one of claims 8 to 10, wherein the perindopril arginine used for the preparation of the granulation liquid is crystalline perindopril arginine, in particular perindopril arginine in a polymorphic form, or perindopril arginine in amorphous form.

12. Pharmaceutical composition comprising
a.) granules comprising perindopril arginine, preferably granules comprising perindopril arginine according to any one of claims 1 to 7,
b.) optionally one or more additional active ingredients, and
c.) optionally one or more auxiliary pharmaceutically acceptable excipients.

13. The pharmaceutical composition of claim 12, wherein the pharmaceutical composition comprises
a.) granules comprising perindopril arginine, preferably granules according to any one of claims 1 to 7,
b.) one or more additional active ingredients, the one or more additional active ingredients being one or more additional active ingredients selected from the group consisting of indapamide and pharmaceutically acceptable salts thereof, amlodipine and pharmaceutically acceptable salts thereof, acetylsalicylic acid and pharmaceutically acceptable salts thereof, bisoprolol and pharmaceutically acceptable salts thereof, atorvastatine and pharmaceutically acceptable salts thereof, and rosuvastatine and pharmaceutically acceptable salts thereof,
c.) optionally one or more auxiliary pharmaceutically acceptable excipients.

14. The pharmaceutical composition of any of claims 12 to 13, wherein the pharmaceutical composition is in the form of a tablet or a capsule, preferably in the form of a tablet.

15. The granules of any one of claims 1 to 7 or the pharmaceutical composition of any one of claims 12 to 14, for use in the treatment of cardiovascular diseases and cardiovascular conditions, especially for use in the treatment of hypertension and heart failure.
